(19) Europäisches Patentamt
European Patent Office
Office européen des brevets

(11) **EP 3 337 896 B1**

(12) **EUROPEAN PATENT SPECIFICATION**

(45) Date of publication and mention
of the grant of the patent:
**23.10.2019 Bulletin 2019/43**

(51) Int Cl.:
*C12N 15/10* (2006.01)   *G01N 33/50* (2006.01)

(21) Application number: **16756680.1**

(86) International application number:
**PCT/EP2016/069803**

(22) Date of filing: **22.08.2016**

(87) International publication number:
**WO 2017/029414 (23.02.2017 Gazette 2017/08)**

(54) **AN IN VITRO 3D CELL CULTURE MODEL-BASED TUMOR RELAPSE ASSAY**

IN-VITRO-3D-ZELLKULTURMODELLBASIERTER TUMORRÜCKFALLASSAY

DOSAGE DE RÉCIDIVE TUMORALE BASÉ SUR UN MODÈLE DE CULTURE CELLULAIRE 3D IN VITRO

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR

(30) Priority: **20.08.2015 GB 201514864**

(43) Date of publication of application:
**27.06.2018 Bulletin 2018/26**

(73) Proprietor: **Insphero AG
8952 Schlieren (CH)**

(72) Inventors:
• **KELM, Jens M.
8610 Uster (CH)**
• **LICHTENBERG, Jan
8103 Unterengstringen (CH)**

(74) Representative: **Michalski Hüttermann & Partner
Patentanwälte mbB
Speditionstraße 21
40221 Düsseldorf (DE)**

(56) References cited:
**EP-A1- 2 796 873**

• **MAS CHRISTOPHE ET AL: "Antitumour efficacy
of the selumetinib and trametinib MEK inhibitors
in a combined human airway-tumour-stroma lung
cancer model", JOURNAL OF BIOTECHNOLOGY,
vol. 205, 20 January 2015 (2015-01-20), pages
111-119, XP029240258, ISSN: 0168-1656, DOI:
10.1016/J.JBIOTEC.2015.01.012**
• **MARIA VINCI ET AL: "Advances in establishment
and analysis of three-dimensional tumor
spheroid-based functional assays for target
validation and drug evaluation", BMC BIOLOGY,
BIOMED CENTRAL, LONDON, GB, GB, vol. 10, no.
1, 22 March 2012 (2012-03-22) , page 29,
XP021125315, ISSN: 1741-7007, DOI:
10.1186/1741-7007-10-29**

**Description**

**Field of the invention**

The present invention relates to methods for determining the potential of a tumor to become resistant to a given anti-cancer agent.

One of the most pressing problems in the treatment of cancer is tumor relapse. Current *in vitro* assays based on monolayer cultures do not provide the required time frame to study tumor recurrence.

Although many tumors regress in response to neoadjuvant chemotherapy, residual tumor cells are detected in most cancer patients post-treatment. These residual tumor cells are thought to remain dormant for years before resuming growth, resulting in tumor recurrence. Considering that recurrent tumors are most often responsible for patient mortality, there exists an urgent need to study signaling pathways that drive tumor dormancy/recurrence.

Current tumor relapse assays in monolayer cultures are based on the re-appearance of cell colonies as monolayer on cell culture surfaces. These assays have, under particular circumstances, shortcomings as regards, inter alia, precision and reproducibility.

Mas et al., Journal of Biotechnology, Vol:205,Page(s):111 - 119, discuss that in a 3D microenvironment tumour cells expand by forming nodules, mimicking a human lung cancer feature. They tested the antitumour potential of the investigational MEK inhibitors selumetinib and trametinib. As primary endpoint, changes in tumour size were assessed by fluorescence measurements. Tumours showed a reduced growth in response to the MEK inhibitors, but halting the selumetinib dosing resulted in tumour relapse.

Vinci et al, Advances in establishment and analysis of three-dimensional tumor spheroid-based functional assays for target validation and drug evaluation, BMC BIOLOGY, Vol:10,Nr:1,Page(s):29 developed a toolkit of reproducible three-dimensional tumor spheroid models for dynamic, automated, quantitative imaging and analysis that are compatible with routine high-throughput preclinical studies. Highly malignant human tumor cells were selected to exemplify therapeutic effects of three specific molecularly-targeted agents. Fully automated analysis using a Celigo cytometer was validated for tumor spheroid growth and invasion against standard image analysis techniques.

It is one object of the present invention to provide an assay which allows detecting and targeting tumor relapse with good precision and high reproducibility.

It is another object of the present invention to extend the possibilities that current tumor relapse methods offer.

**Embodiments of the invention**

These and further objects are met with methods and means according to the independent claims of the present invention. The dependent claims are related to preferred embodiments. It is yet to be understood that value ranges delimited by numerical values are to be understood to include the said delimiting values.

**Summary of the invention**

Before the invention is described in detail, it is to be understood that this invention is not limited to the particular component parts of the devices described or process steps of the methods described as such devices and methods may vary. It is also to be understood that the terminology used herein is for purposes of describing particular embodiments only, and is not intended to be limiting. It must be noted that, as used in the specification and the appended claims, the singular forms "a," "an" and "the" include singular and/or plural referents unless the context clearly dictates otherwise. It is moreover to be understood that, in case parameter ranges are given which are delimited by numeric values, the ranges are deemed to include these limitation values.

According to one embodiment of the invention, a tumor relapse assay method is provided, which method comprises

a) Providing a 3-dimensional cell culture or tissue comprising immortalized, malignant, cancerous and/or neoplastic cells

b) Exposing the 3-dimensional cell culture or tissue to at least one anti-cancer agent,

c) Interrupting the exposure,

b') exposing the 3-dimensional cell culture or tissue at least one further time with the same or at least one different anti-cancer agent, and

d) Determining the effect of said exposure and interruption on the 3-dimensional cell culture or tissue.

3-dimensional cell cultures or tissues are preferably cultured in a particular vessel, e.g. a microreaction vessel or a well of a microtitre plate. Exposing the 3-dimensional cell culture or tissue to at least one anti-cancer agent means, for example, that said anti-cancer agent means is added to that vessel, e.g., by means of a suitable pipette, pipetting robot or dispenser.

Interrupting the exposure means, for example, that the medium surrounding the 3-dimensional cell culture or tissue is removed from the vessel, and new medium devoid of said anti-cancer agent is added.

This embodiment takes particular benefit of the 3-dimensional cell culture or tissue. The inventors have realized that the conventionally used 2-dimensional cell culture assays frequently fail to correlate with *in vivo* observations.

Although it is commonly accepted that tumor sensitivity or resistance to chemotherapeutic agents is genetically determined, less attention is generally devoted to the fact that such resistance may also be driven by the tumor microenvironment. The inventors have shown that metabolic gradients and the extracellular matrix can influence nutrient availability to various segments of a solid tumor, and also limit the ability to administer an effective dose of chemotherapeutic agent. These biological and therapeutic gradients result in the formation of phenotypically different tumor cell subpopulations exposed to a variety of therapeutic to sub-therapeutic drug doses *in vivo.* Thus, the inventors found that 3-dimensional tumor microtissues, or multicellular tumor spheroids, provide a more representative, organotypic model for assessment of tumor growth. They contain layers of cells that exhibit more *in vivo*-like size- and gradient-dependent proliferation and viability profiles.

In contrast thereto, 3-dimensional cultures allow to recapitulate the native tumor microenvironment.

For these reasons, cells in a 3-dimensional cell culture behave more physiologically than cells in 2-dimensional cell culture, because they can better establish intercellular communication pathways as well as extracellular matrices. Furthermore, a 3-dimensonal tissue better reflects physicochemical conditions in a true tissue or organ, because it better simulates diffusion gradients of both gases, like oxygen, and chemical agents. Further, it better simulates penetration barriers for larger components.

One other aspect is that, in 2-dimensional cell cultures, cancer stem cells do not find the specific tissue niches for maintaining their cancer stem cell phenotype. The inventors found, surprisingly, that in a 3-dimensional cell culture according to the invention, the relapse properties of cancer stem cells can for the first time be suitably investigated.

Yet another advantage is that, while 2-dimensional cell cultures allow mere quantification of resistant colonies (endpoint measurement), a 3-dimensional cell culture according to the invention allows to observe the kinetic of a tumor relapse. The inventors have suggested this kinetic approach for the first time, and discuss the many surprising advantages emanating therefrom.

Still another advantage is that a 3D microtissue also allows to tailor a cancer model which faithfully reflects a true tumor, by combining drug resistant and sensitive cells within the same tissue to specifically monitor drug effects on either cell population.

Further, compared to 2-dimensional cell cultures, 3-dimensional cell cultures or tissues have a significantly higher lifetime. While 2-dimensional cell cultures comprising non-immortalized primary cells have an assay lifetime of 3 - 7 days, 3-dimensional cell cultures or tissues have a lifetime of up to 30 days.

For that reason, long term effects of exposure to the anti-cancer agents can much better be investigated than in 2-dimensional cell cultures. Furthermore, kinetics of the respective responses can better be analyzed.

3-dimensional cell cultures or tissues are therefore more suitable models for toxicity screening as well as for resistances assays than 2-dimensional cell cultures.

In its plainest and simplest form, the method according to the invention allows to investigate whether or not, after or during an interruption of the exposure, a tumor relapses from a first line treatment, and to what extent. Further, this method allows to determine whether or not a relapse is dependent of the concentration of the first line treatment.

In the first variant of this embodiment, the method according to the invention allows to investigate whether or not, after an interruption of the exposure to a first line treatment, a tumor that has, or has not, relapsed from a first line treatment, can be controlled again, or shrunk further, with the first line treatment. Further, this method allows to determine whether or not the measured response is dependent of the concentration of the first line treatment.

In the second variant of this embodiment, the method according to the invention allows to investigate whether or not, after an interruption of the exposure to a first line treatment, a tumor that has, or has not, relapsed from a first line treatment, can be controlled again, or shrunk further, with a second line treatment. Further, this variant can be used to screen for a suitable 2nd line treatment, i.e., at least one different anti-cancer agent. Further, this method allows to determine whether or not the measured response is dependent of the concentration of the second line treatment.

In principle, the different treatment schemes according to the invention are shown in the following:

**Table 1**

| Issue to be investigated | Does the tumor relapse from the 1st line treatment ? Is relapse concentration-dependent ? | Can tumor relapse be controlled again with the 1st line treatment, or is tumor resistant thereto ? | Can tumor relapse after 1st line treatment can be controlled again with a 2nd line treatment, and if so, which one ? |
|---|---|---|---|
| 1st exposure (step b) | Anti-cancer agent A | Anti-cancer agent A | Anti-cancer agent A |
| Interruption (step c) | | | |
| 2nd exposure (step b') | n/a | Anti-cancer agent A | Anti-cancer agent B |

Again, note that steps c and b' can be repeated one or more times.

Preferably, the exposure time to the anti-cancer agent can last anything between hrs and weeks. Preferably, the interruption of thee exposure can be accomplished by replacing the medium comprising the anti-cancer agent by agent-free medium. Preferably, the interruption of the exposure can last anything between days and months.

The immortalized, malignant, cancerous and/or neoplastic cells that are comprised in the 3-dimensional cell culture or tissue can be derived from, e.g.,

- Tumor cell lines
- Immortalized cell lines
- Tumor xenografts
- Patient-derived xenografts, and/or
- Patient biopsies

Cells derived from tumor cell lines can, *inter alia,* be used for basic research, or for characterizing and/or screening different types of drugs on their

Cells from tumor xenografts are a derivation thereof, in that cells from tumor cell lines have first been used to establish a xenograft in a susceptible host, e.g., a tumor mouse. In a second step, cells from that xenograft are derived to fabricate the 3-dimensional cell culture or tissue. Because of the intermediate xenograft stadium, these cells behave in a more physiological manner than if directly derived from a tumor cell line.

Patient-derived xenografts (PDX) are created when cancerous tissue from a patient's primary tumor is implanted directly into an immunodeficient mouse. PDX models are providing solutions to the challenges that researchers face in cancer drug research such as positive tumor responses in mouse models but not translating over when the study is implemented in humans.

Cells derived from patient biopsies can deliver a faithful reproduction of the tumor of a particular patient, and can thus be used for investigating patient specific tumor relapse, for example in order to develop a personalized cancer therapy.

According to a preferred embodiment, the 3-dimensional cell culture or tissue also comprises at least one cell type selected from the group consisting of:

- Immune cells

- Stroma cells

- Fibroblasts

- Cancer stem cells

- Immortalized, malignant, cancerous and/or neoplastic cells that are known to be resistant or sensitive to a given anti-cancer agent,

Using immune cells in such 3-dimensional cell culture or tissue has a particular benefit because effects of the innate immune system can also be investigated. This is particularly useful in case an anti-cancer agent is investigated which co-acts with the immune system i.e., an immunotherapeutic agent, like an immune checkpoint inhibitor (see infra). These drugs toughen up the immune system to actively combat tumor cells. Therefore, to test their efficacy in vitro, and to investigate potential relapses, these agents require the presence of immune cells.

Examples of suitable immune cells that can be used for such co-culture encompass, inter alia, tissue resident leukocytes, like macrophages (e.g., stellate macrophages/kupffer cells), CD8[+] T cells, natural killer cells, natural killer T cells, and/or regulatory T cells.

Using connective tissue components, like stroma cells or fibroblasts, may help recreating the important tumor microenvironment in vitro, and thus increase the quality of the 3-dimensional cell culture or tissue, and this improve the predictive value of such culture or tissue for assays or screening purposes.

Cancer stem cells (CSCs) are cancer cells that possess characteristics associated with normal stem cells. Cancer stem, calls sometimes share a feature with normal somatic stem cells in that they are naturally resistant to chemotherapeutic agents, because they express various pumps that pump out drugs, plus DNA repair proteins. They also have a slow rate of cell turnover. CSC are thus able to survive first lien cancer treatment and then repopulate the tumor, causing a relapse.

Co-culturing immortalized, malignant, cancerous and/or neoplastic cells with cancer stem cells can thus help to develop therapies which affect both the primary tumor and, at the same time, inhibit cancer stem cells.

The same rationale applies to the co-culture of a given immortalized, malignant, cancerous and/or neoplastic cell type with another immortalized, malignant, cancerous and/or neoplastic cell type that is known to be resistant or sensitive to a given anti-cancer agent,

Preferably, the one or more anti-cancer-agents are selected from the group consisting of:

- cytotoxic and/or chemotherapeutic agents
- targeted drugs
- immunotherapeutic agents,
- radiation

and/or combinations thereof. As used herein, the term "cytotoxic and/or chemotherapeutic agent" refers to drugs that interact with tumor cell division, thereby killing cells that proliferate quickly.

As used herein, the term "targeted drugs" refers to drugs that specifically bind to a ligand, receptor or cellular antigen, like target specific antibodies, or fragments or derivatives therof, or tyrosine kinase inhibitors. The term relates mainly to antibodies, and fragments or derivatives thereof, like receptor Fc fusion peptides, and other biologicals that have high target specificity, like antibody mimetics. The term also encompasses other antibody-based agents like antibody drug conjugates and immunotoxins, as well as bi- or trispecific antibodies.

As used herein, the term "immunotherapeutic agents" refers to agents that support the body's immune system in fighting cancer, in particular

- Immune checkpoint modulators (ICM) and inhibitors (ICI), like antibodies antibodies binding CTLA4, PD1, PD-L1, LAG 3, TIM3 and/or OX40
- antibodies or fusion proteins evoking antibody-dependent cellular cytotoxicity (ADCC) or complement-dependent cytotoxicity (CDC)
- Bispecific antibodies or fusion proteins which bind tumor antigens and attract T cells, NK cells or dendritic cells and
- Dendritic cell (DC) therapies
- Modified T-cells with chimeric T cell receptors (CAR-T), and/or
- Cancer vaccines.

In this context, it is particularly preferred to use a 3-dimensional cell culture or tissue that comprises tumor cells and immune cells, as set forth above.

It is known that tumors recurring after neoadjuvant therapy often acquire a phenotype radically different from that of their originating primary tumors. This new phenotype allows them to escape a host-derived innate immune response mediated by cells of the immune system. This resistance can for example be due to the secretion of ligands that bind to immunce checkpoint inhibitors, thus pacifying the immune system to no longer attack the tumor.

The present invention enables the screening for anti-cancer agents which restore the tumor's sensitivity to the neoadjuvant therapy, reactivate the immune system to act against the tumor, or which themselves have activity against said types of tumor.

According to another preferred embodiment, the determination of cell proliferation and/or cell viability is at least one selected from the group consisting of:

- Size determination of the 3-dimensional cell culture or tissue

- Quantification of internal reporter gene expression

- Determination of the intracellular ATP content.

- Determination of particular biomarkers

This embodiment takes particular benefit of the 3-dimensional cell culture or tissue. In 2-dimensional cell cultures or tissues, cell proliferation cannot be measured in real time. In such cultures or issues, an endpoint measurement can only be made by counting the colonies in the cell culture.

Internal reporter gene expression such as fluorescent proteins, luciferases or peroxidases, can be measured with suitable readers, e.g., fluorescence or luminescence readers or transillumination readers.

Intracellular ATP as a measure for metabolic activity can for example be determined by using CellTiter-Glo® Luminescent Cell Viability Assay (Promega; Madison, USA). Details of such approach are shown elsewhere herein.

Biomarkers can, e.g., be tumor markers, like PSA, AFP (Liver Cancer), BCR-ABL (Chronic Myeloid Leukemia), BRCA1/BRCA2 (Breast/Ovarian Cancer), BRAF V600E (Melanoma/Colorectal Cancer), CA-125 (Ovarian Cancer), CA19.9 (Pancreatic Cancer), CEA (Colorectal Cancer), EGFR (Non-small-cell lung carcinoma), HER-2 (Breast Cancer), KIT (Gastrointestinal stromal tumor), PSA (Prostate Specific Antigen) (Prostate Cancer), S100 (Melanoma), or epigenetic markers, These biomarkers can be measured, e.g., with Elisa, HPLC, MS, Immunohistochemistry or FACS, or with hybridization-based techniques (PCR, rtPCR etc)

According to another preferred embodiment, the size determination of the 3-dimensional cell culture or tissue refers to at least one parameter selected from the group consisting of:

- diameter

- perimeter

- volume

- area of an optical cross section

The size can thus either be a parameter that has directly been measured, or a parameter which has been calculated on the basis of such measurements.

Preferably, the size determination of the 3-dimensional cell culture or tissue is carried out by means of an imaging device.

One example of such imaging device is the Cell$^3$iMager manufactured by, InSphero AG, Schlieren, CH. It allows analysis of spheroids by scanning multi-well plates in a bright-field. It computes estimated values based on spheroid size and density, together with spheroid number and area in the each well. With easy and efficient operability, its vibration-free design protects cells from damage. An excellent application is also available to determine spheroid proliferation over time and to measure the granular distribution in 3D culture.

According to another preferred embodiment the determination of the effect of the anti-cancer agent exposure on the 3-dimensional cell culture or tissue is determined by

- kinetic measurements of cell proliferation and/or cell viability, and /or
- endpoint measurements of cell proliferation and/or cell viability.

The term "kinetic measurement" (also called "real time measurement") is related to those types of measurement in which a given parameter is monitored continuously or frequently during the exposure of the 3-dimensional cell culture or tissue to the at least one anti-cancer agent, including times of interruption of the exposure. Preferably, said kinetic measurement of cell proliferation and/or cell viability is a size determination, e.g., diameter, volume or area of an optical cross section.

The term "endpoint measurement" is related to those types of measurement in which a given parameter is measured at a predetermined point of time, which is often considered to mark the end of a given process. Preferably, said endpoint measurement of cell proliferation and/or cell viability is a size determination, e.g., e.g., diameter, volume or area of an optical cross section, or a measurement of intracellular ATP.

In one preferred embodiment of the tumor relapse assay method, the cell proliferation and/or viability of the 3-dimensional cell culture or tissue which has been exposed to the at least one anti-cancer agent in an interrupted fashion ("pulsed treatment group") is compared to

a) cell proliferation and/or viability of at least one 3-dimensional cell culture or tissue which has been exposed to at least one anti-cancer agent without any interruption ("continuous treatment group"), and/or

b) cell proliferation and/or viability of at least one 3-dimensional cell culture or tissue which has not been exposed to the at least one anti-cancer agent ("untreated control group").

Preferably, the cell proliferation and/or viability of two or more 3-dimensional cell cultures or tissues which underwent different exposure protocols are determined at the same time after treatment onset.

Such comparison may take place by quotient formation of the respective sizes, e.g., diameter, volume or area of an optical cross section. While the time after treatment onset should be identical, the total exposure time to the anti-cancer agent can be different in the different 3-dimensional cell cultures or tissues. It will for example be higher in the "continuous treatment group" than in the pulsed treatment group, for example.

In another preferred embodiment, the method comprises the determination of a time to tumor relapse by forming a quotient of:

a) the cell proliferation and/or viability of at least one 3-dimensional cell culture or tissue which has been exposed to the at least one anti-cancer agent in an interrupted fashion ("pulsed treatment group"), and

b) the cell proliferation and/or viability of at least one 3-dimensional cell culture or tissue which has been exposed to at least one anti-cancer agent without any interruption,

and determining a time after onset of exposure to the anti-cancer agent at which said quotient exceeds a given threshold.

Such time to tumor relapse can for example be defined as the time at which the cell culture or tissue that underwent interrupted exposure as gained 10 % size increase compared to the cell culture or tissue that underwent interrupted exposure.

Other preferred thresholds are 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 and/or 100 % size increase.

Particularly preferred thresholds are 10, 20, 30, 40, 50, 60, 70, 80, 90, and/or 100 % size increase.

In another preferred embodiment, the method comprises the determination of a tumor relapse index ("Rel"). Preferably, the determination of the tumor relapse index comprises the following steps:

a) determine the cell proliferation and/or viability of at least

(i) one 3-dimensional cell culture or tissue which has been exposed to the at least one anti-cancer agent in an interrupted fashion ("pulsed treatment group"),

(ii) one 3-dimensional cell culture or tissue which has been exposed to the at least one anti-cancer agent without any interruption ("continuous treatment group"), and

(iii) one 3-dimensional cell culture or tissue which has not been exposed to the at least one anti-cancer agent ("untreated control group").

at a given point of time,

b) determine

(i) the difference (e.g., $d_2$) between the cell proliferation and/or viability of at least one 3-dimensional cell culture or tissue in the continuous treatment group and at least one 3-dimensional cell culture or tissue in the pulsed treatment group, and

(ii) the difference (e.g., $d_1$) between the cell proliferation and/or viability of at least one 3-dimensional cell culture or tissue in the pulsed treatment group and at least one 3-dimensional cell culture or tissue in the untreated control group, and

c) form a quotient out of the two differences (e.g., $d_1/d_2$) to deliver a tumor relapse index.

Preferably, the tumor relapse indices can be determined for different inhibitory concentrations of the respective anti-cancer agents, like, e.g., $IC_{20}$ and/or $IC_{50}$ to enable comparability in between different sets of compounds and compound combinations. Likewise preferably, the parameter that is measured for cell proliferation is size (e.g., diameter, volume or area of an optical cross section).

The relative tumor relapse indices allow an estimation whether the growth of the pulsed treatment group is closer to that of the untreated group or to that of the continuously treated group.

In one embodiment, a high value for $d_1$ signifies that there is a significant difference between the growth of the untreated control group and the growth of the pulsed treatment group, suggesting that even the pulsed treatment has good efficacy and does not result in resistance or relapse. Likewise, a small value for $d_2$ signifies that there is little difference between the growth of the pulsed treatment group and the growth of the continuously treated group, suggesting that even the pulsed treatment has good efficacy and does not result in resistance or relapse. In this embodiment, a high value for $d_1$ and small value for $d_2$ result in a high relative tumor relapse index, which stands, inter alia, for little risk of or relapse, while, on the contrary, a small value for $d_1$ and high value for $d_2$ result in a small relative tumor relapse index, which stands, inter alia, for high risk of or relapse.

It is important to understand that the relative tumor relapse indices are also concentration-dependent. For example, at IC50 a drug may have a more sustainable effect, resulting in little risk of relapse, while at IC20, the effect may be less sustainable, and more prone to relapse.

In one embodiment, a stratification according to ReI can be as follows:

**Table 2**

| ReI = 10 - ∞ | Low probability of tumor recurrence and high impact on tumor growth rate at a given concentration and treatment scheme |
|---|---|
| ReI = 1-10 | Moderate probability of tumor recurrence and/or reduced tumor growth rate at a given concentration and treatment scheme |
| ReI = <1: | High probability of tumor recurrence and/or minor impact on tumor growth at a given concentration and treatment scheme. |

In another preferred embodiment, the method according to the invention comprises the calculation of a growth constant of at least one 3-dimensional cell culture or tissue.

One preferred way to determine a growth constant is to create a quotient of cell proliferation, e.g., size (e.g., diameter, volume or area of an optical cross section) over time after treatment onset. In such way, growth constants for the continuous treatment group, the pulsed treatment group and the untreated control group can be formed.

According to another preferred embodiment, the determination of the cell proliferation and/or viability of a 3-dimensional cell culture or tissue treated in a particular manner comprises the determination of the cell proliferation and/or viability of two or more 3-dimensional cell cultures or tissues treated in the same manner, and forming a statistical mean or median for proliferation and/or viability.

Preferably, the method further comprises a step of determining an inhibitory concentration of the at least one anti-cancer agent on the a 3-dimensional cell culture or tissue.

**Range finding**

Particularly preferred, the inhibitory concentration is $IC_{50}$ and/or $IC_{20}$.

The term "$IC_{50}$" relates to the half maximal inhibitory concentration. The term "$IC_{20}$" relates to a 20%-*inhibitory* concentration. Both parameters are a measure of the effectiveness of an agent in inhibiting a specific biological function, e.g., tumor cell proliferation.

According to another preferred embodiment, the 3-dimensional cell culture or tissue has been produced in a hanging drop culture system or a low adhesion well culture system.

One example of such hanging drop culture system is the GravityPLUS™ Hanging Drop System manufactured by InSphero AG, Schlieren, CH. This system allows scaffold-free re-aggregation of single cells into functional 3D microtissues, because it avoids the provision of liquid/solid interfaces to which cells can adhere.

One example of such low adhesion well culture system is the GravityTRAP™ ULA Plate System manufactured by InSphero AG, Schlieren, CH. These plates comprise non-adhesively coated wells which allow the formation of 3-dimensional cell cultures or tissues by avoiding adherence of the cells to the solid interface.

According to another aspect of the invention, in the described method

a) at least two 3-dimensional cell cultures or tissues of similar or identical composition are used in such way that each cell culture or tissue is exposed to a different anti-cancer agent, or

b) at least two 3-dimensional cell cultures or tissues of different composition are used in such way that both cell cultures or tissues are exposed to the same anti-cancer agent.

The first embodiment allows to compare different anti-cancer agents, and the respective risks of relapses, with one another. This approach can easily be scaled up using state of the art high throughput techniques, like multiwall plates, suitable plate readers and imagers, like the ones discussed herein. In such way, drug libraries can be screened through in search of a drug against which the respective tumor model is least likely to relapse, or develop a resistance.

This again may help to find a patent specific cancer therapy, or to create benchmarks for particular anti-cancer drugs regarding their risk to become subject of relapses.

The second embodiment allows to compare different 3-dimensional cell cultures or tissues, in particular on their potential to develop relapses, and helps to investigate the reasons for relapse, and the effect of external factors, like non-tumor cells, thereon.

Preferred parameters compared in the two above approaches are growth constant, tumor relapse index, time to tumor relapse, and/or inhibitory concentration, as discussed herein.

According to yet another preferred embodiment of the present invention, the method further comprises exposure of the 3-dimensional cell culture or tissue to one or more immune cells.

As discussed elsewhere herein, using immune cells in a tumor relapse assay according to the present invention such 3-dimensional cell culture or tissue has a particular benefit because effects of the innate immune system can also be investigated. This is particularly useful in case an anti-cancer agent is investigated which co-acts with the immune system i.e., an immunotherapeutic agent, like an immune checkpoint inhibitor (see *supra*). These drugs toughen up the immune system to actively combat tumor cells. Therefore, to test their efficacy *in vitro,* and to investigate potential relapses, these agents require the presence of immune cells.

As discussed already, the said immune cells can already be in the 3-dimensional cell culture or tissue, thereby exposing the latter to one or more immune cells.

In yet another approach, immune cells which are not per se part of the 3-dimensional cell culture or tissue can be added to the latter, e.g., in the reaction vessel, prior to, after or together with the anti-cancer agent, which is for example an immunotherapeutic.

Suitable cells that van be used for this approach encompass, but are not limited to, PBMC (peripheral blood mononuclear cells)

According to another aspect of the present invention, the use of a tumor relapse assay method according to the invention for at least one purpose selected from the group consisting of:

- comparing different anti-cancer agents, and the respective risks of relapses,
- screening drug libraries in search of anti-cancer agents with low risk of becoming subject of a relapse,
- screening drug libraries in search of anti-cancer agents which have efficacy against a tumor that has already relapsed after therapy with another anti-cancer agent,
- screening drug libraries in search of anti-cancer agents which restore the sensitivity of a tumor that has already relapsed after therapy with another anti-cancer agent,
- finding a patent-specific anti-cancer therapy,
- creating benchmarks for particular anti-cancer drugs regarding their risk to become subject of relapses,
- comparing different 3-dimensional cell cultures or tissues on their potential to develop relapses after therapy with an anti-cancer agent, and/or
- investigating the reasons for tumor relapse after therapy with an anti-cancer agent.

is provided.

## Experiments and Figures

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

## Generation of 3D microtissues according to the present inventions

Generation of 3D microtissues is a straightforward process that works with the vast majority of cell types capable of forming tissues *in vivo.* The process overview illustrates the formation tumor cell line microtissues.

**Materials required**

1. Cells as defined as source material hereinabove
2. 3D InSight™ Tumor Microtissue Media Kit (InSphero, cat. no. CS-17-001-01 -includes 3D InSight™ Tumor Re-aggregation Medium (CS-07-111-02) and 3D InSight™ Tumor Maintenance Medium (CS-07-112-01)
3. Inverted microscope with a $5\times$ objective or a $10\times$ long distance objective with a distance ring (see also Appendix 1)
4. Cell counter, e.g. Neubauer chamber
5. 8- or 12-channel pipette (e.g. Viaflo 10.0-300.0 μl, Integra Biosciences, InSphero, cat. no. IS-001-01)
6. Medium reservoir for multichannel pipettes
7. For microscopic observation of the full GravityPLUS™ Plate, an additional GravityPLUS™ bottom plate is recommended
8. Microplate centrifuge
9. Humidified $CO_2$ incubator

**Preparation**

1. Wipe the GravityPLUS™ Plate bag with 70% EtOH before opening.
2. Carefully open the bag under sterile working conditions and take out the GravityPLUS™ Plate assembly.
3. Prepare a reservoir (e.g. a 15 cm diameter petri dish) with 20 ml 0.5x PBS.
4. Open the bag containing humidifier pads. Using the tweezers, remove one humidifier pad and place it in the dedicated reservoir containing the 0.5x PBS.
5. Wait until the humidifier pad is completely soaked with PBS (approx. 5 min).
6. While pad is soaking, open the GravityPLUS™ Plate package and remove the raster plate
7. Place the soaked humidifier pad in the bottom plate of the GravityPLUS™ Plate.
8. Trypsinize cells expanded in cell-culture flasks according to your standard protocol.
9. Count the cells. Prepare a cell suspension. Recommended cell concentration: For long-term growth profiling start with low cell numbers (250-500 cells per drop). If non-proliferating cells or rapid production of larger microtissues is required then start with 2500-25,000 cells/40 μl.
10. Gently deliver 40 μl of cell suspension into each well of the GravityPLUS™ Plate. It is easy to ensure tight contact between the pipette tip and the well inlet
11. Place the lid on the GravityPLUS™ Plate.
12. Place the GravityPLUS™ Plate assembly in a humidified $CO_2$ incubator at 37°C.
13. Assess microtissue formation regularly. After 4 days in culture most cell types re-aggregate and form a compact spheroid.

**3D Microtissue production**

HCT-116 (DSMZ no. ACC 581) cells were expanded as monolayer in cell culture flasks using VLE RPMI 1640 medium (Biochrom GmbH, Germany) supplemented with 10% fetal bovine serum (FBS Superior, Biochrom GmbH, Germany), 100 unit/ml penicillin and 100 μg/ml streptomycin at 37°C and 5% $CO_2$. To produce HCT-116 3D InSight™ colorectal cancer microtissues, cells were detached from the 2D culture flask using trypsin/EDTA and seeded into 96-well hanging drop GravityPLUS™ plates. After microtissue formation, spheroids were transferred into GravityTRAP™ plates for further experiments. Medium was refreshed at days 4, 6 and 8 post-transfer to assay plates.

**Compound treatment of HCT 116 3D InSight™ microtissues**

Compounds (staurosporine, taxol, doxorubicin) to treat HCT-116 microtissues in GravityTRAP™ plates were purchased from Enzo Life Sciences (Lausen, Switzerland) and dissolved in DMSO. From the stock solutions, two-fold serial dilutions in DMSO were prepared for each compound, leading to 200x concentrated working dilutions. The assayed concentration range of staurosporine was 2 to 1'000 nM, for taxol it was 1.6 nM to 800 nM and for doxorubicin the range was between 0.8 nM and 400 nM.

Microtissues were treated in GravityTRAP™ plates with the indicated compound dilutions (n=4), leading to 0.5% DMSO in the cell culture medium. As a control, microtissues were exposed to 0.5% DMSO without a compound. Upon medium exchange, compounds were re-added where indicated. The treatment was performed over 10 days, however, for 2 tissues per group, the compound exposure was discontinued after 5 days and they were thereafter incubated in medium without compound (n = 2).

IC20 and IC50 values were determined by plotting the tissue size over the compound concentration after incubation over 5 days.

**3D InSight microtissue size profiling**

GravityTRAP™ plates containing treated microtissues were scanned with Cell[3]iMager (Dainippon, Japan) with a resolution of 4800 dpi. Microtissue size was assessed with the software provided by the manufacturer.

Air bubbles interfering with the size analysis were excluded manually, either by adjusting the image section to be analyzed by the software or by cropping the area designated to a microtissue after size measurement. The acquired data was normalized to the non-treated control (S = size of microtissue).

$$Average_{size,norm} = Average(\frac{S_1}{S_{1;control}}; \frac{S_2}{S_{2;control}}; \frac{S_3}{S_{3;control}}) \qquad (1)$$

**Determination of cell viability**

In order to determine the number of viable cells per microtissue, the amount of intracellular ATP as a measure for metabolic activity was determined by using CellTiter-Glo® Luminescent Cell Viability Assay (Promega; Madison, USA). The assay was performed according to the manufacturer's protocol with slight adaptations specific to microtissues. In brief, supernatant was removed and lysis of microtissues initiated by adding 40 $\mu$l diluted CellTiter-Glo Reagent (1:2 in PBS). The cell lysis was enhanced by pipetting up and down prior to transfer into an opaque white assay plate. Relative luminescence (RLU) was recorded with a TECAN™ Infinite 200 microplate reader, with sensitivity set to 1'000 ms. The acquired data was normalized to the non-treated control:

$$Average_{ATP,norm} = Average(\frac{R_1}{R_{1;control}}; \frac{R_2}{R_{2;control}}; \frac{R_3}{R_{3;control}}) \qquad (2)$$

**Results**

**Determination of IC20, IC50 and max. response concentration**

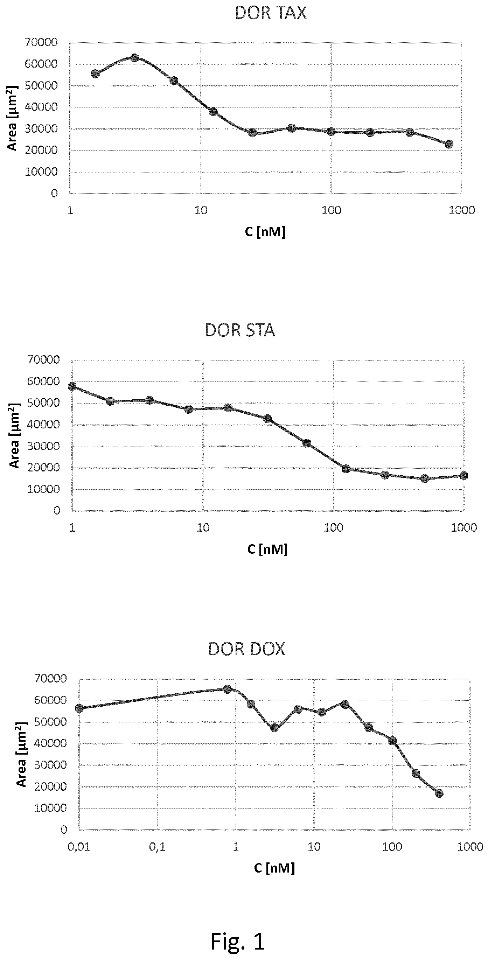

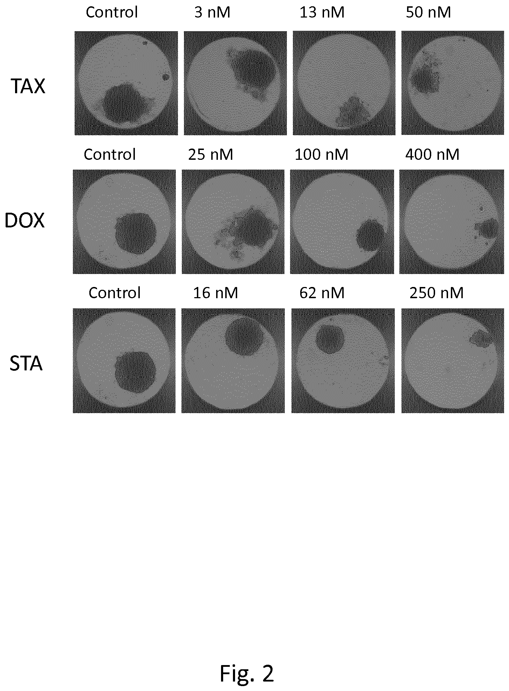

We have tested three reference drugs and monitored growth over time by non-disruptive quantification of size. Size profiling after 5 and 10-days treated with taxol (TAX), doxorubicin (DOX) and staurosporine (STA) displayed dose-depended size differences (Figures 1 and 2). Even with a high degree of tissue debris and dissolving of clear spherical appearance the core tissue borderline was detected (see line around the microtissues in Figure 2). Dose response based on the size (area) of the tumor microtissue were used to define IC20, IC50 and the lowest maximum response concentration [nM].

**Monitoring tumor relapse**

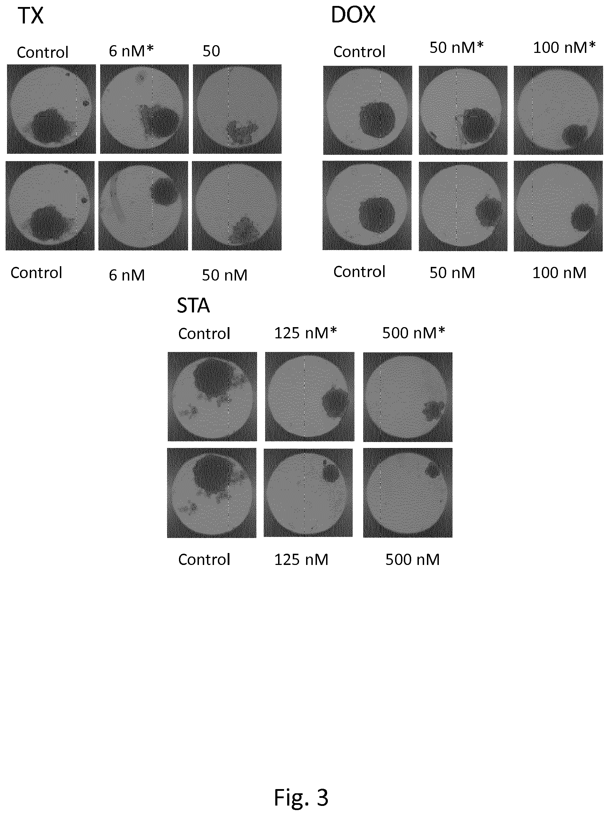

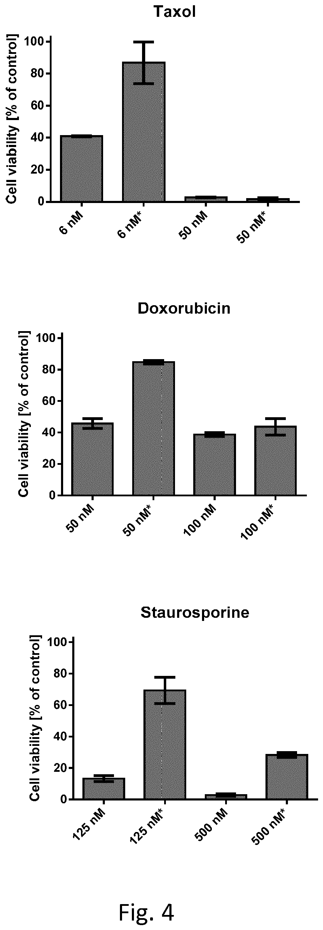

Differences between the drugs were also observed after removing the compound after a 5-day treatment to assess recurrence of tumor. Whereas there was no re-growth observed using the high drug concentrations, tumor re-growth was observed measuring either size or intra-tissue ATP content for lower drug concentrations, as exemplified for 6 nM for taxol, at 50 nM for doxorubicin and 125 nM for staurosporine (Figures 3 and 4). Phenotypic analysis showed that whereas the doxorubicin and staurosporine-treated microtissues regrew symmetrically, the taxol-treated microtissues regrew side-specific at low concentrations (6nM; Figure 3).

The growth curves of continuously treated tumor microtissues and the single-dosed groups confirm the qualitative analysis. High concentrations of the drugs led to a nearly complete tumor removal after 5-days for taxol whereas at lower concentrations tumor started to regrow (Figures 3 and 4). For doxorubicin 50 and 100 nM treated groups were compared. Whereas both concentrations effected growth of the tumor microtissues in a similar way the 50 nM treated group demonstrated a stronger capacity to re-grow after drug removal (Figure 3 and 4). In the case of staurosporine tissue which displayed similar size reduction at 125 nM and 500 nM after 5 days, re-grew substantially as compared to their continuous treated counterparts based on size (Figure 3) and intra-tissue quantification (Figure 4).

**Calculation of the relapse index (ReI) for compound classification**

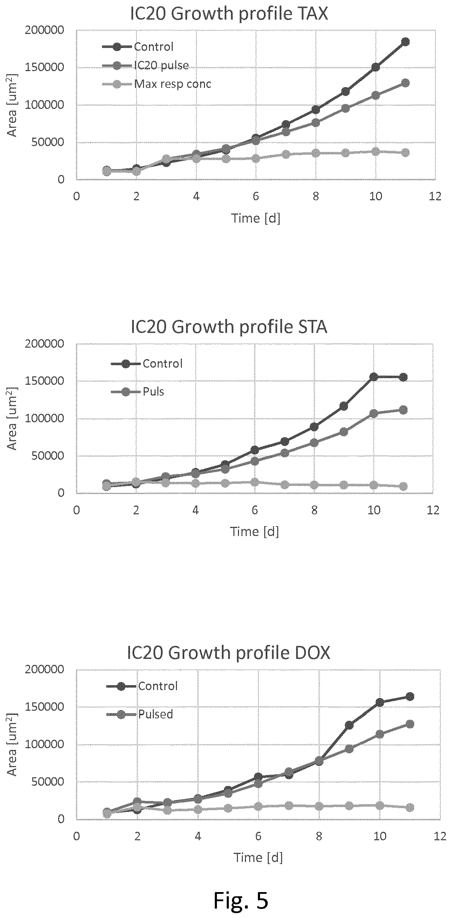

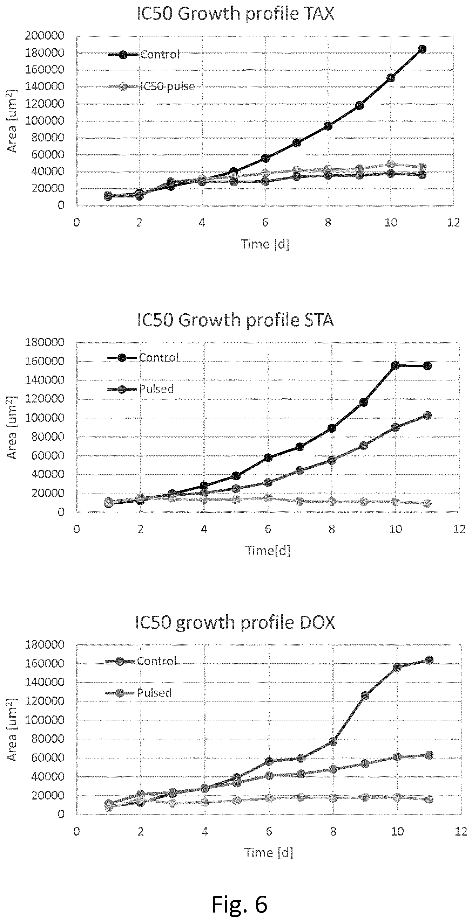

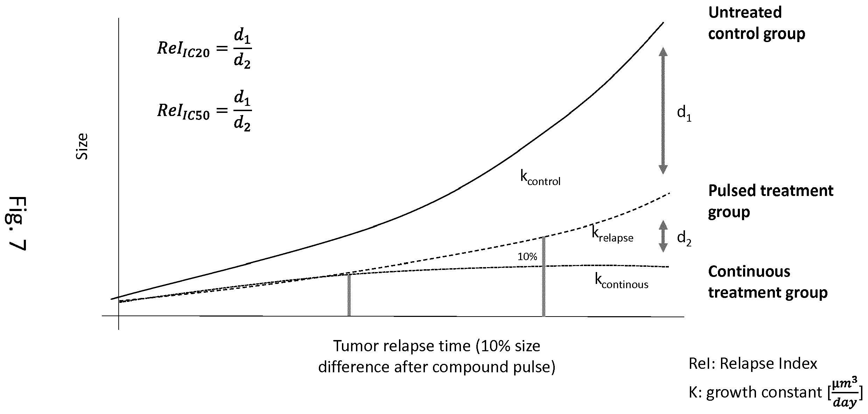

To calculate the tumor relapse index the tumor recurrence growth profiles (area) over 10 days were compared at the respective IC20, IC50 concentration (Figures 5 and 6). To generate the tumor relapse index the growth profiles of the pulsed treatment groups were compared to the growth curves of the vehicle-only treated microtissues and the lowest maximum concentration treated group (Figure 7). The impact of the pulsed drug treatment was set relative to the control and the maximum response of the respective drug (Figures 5 and 6).

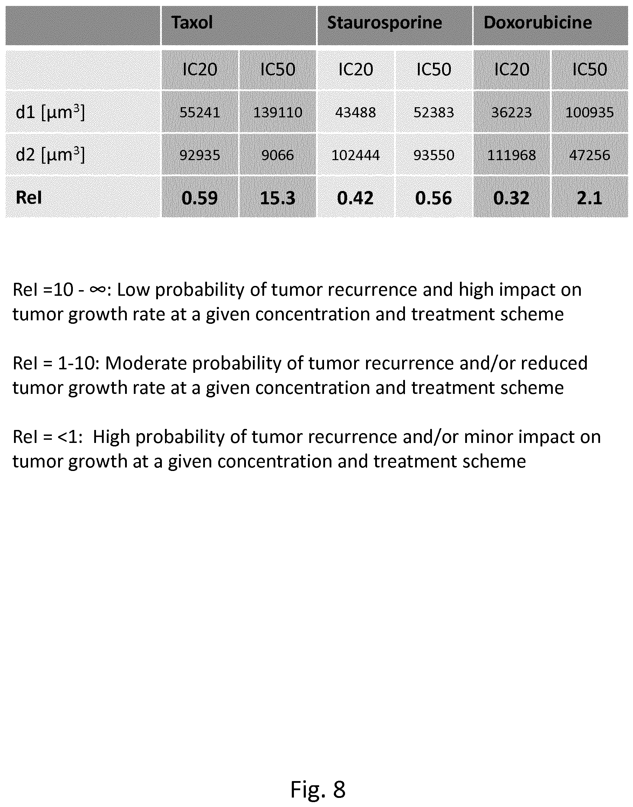

Comparing the growth profiles at IC20 demonstrates that a 5-day treatment did not lead to a severe impact on the growth the microtissues. Consequently, the ReI does not significantly differ between the three compounds tested ranging between 0.32 for doxorubicine and 0.59 for taxol (Fig. 8).

However, comparing the ReI at the respective IC50 concentrations shows clear differences in the ability of the drugs to eliminate the tumor after a 5-day treatment. Whereas for taxol hardly any growth could be observed after the 5-day treatment, for doxorubicin and staurosporine the tumor was still able to grow. In the case of staurosporine, growth occurred even in a similar extent as in the non-treated control culture.

These different responses at IC50 are reflected by the ReI of the three compounds: Taxol has a ReI of 15.3 (26 times higher than at IC20); Doxorubicin has a ReI of 2.1 (6.6 times higher than at IC20) and Staurosporine has a ReI of 0.56 (1.3 times higher than at IC20), (Fig. 8). Further, as shown in Table 2, in one embodiment a ReI of $\geq 10$ means a low probability of tumor recurrence and high impact on tumor growth rate at a given concentration and treatment scheme, while a ReI of < 10 means High probability of tumor recurrence and/or minor impact on tumor growth at a given concentration and treatment scheme.

The tumor relapses index as discussed above thus provides a meaningful benchmark to decide whether a tumor model, e.g., 3-dimensional cell culture or tissue comprising immortalized, malignant, cancerous and/or neoplastic cells, relapses after treatment with one or more anti.-cancer agents. It can thus help to investigate

a) whether a tumor relapses from the 1st line treatment, and , if yes, whether such relapse is concentration-dependent
b) whether a tumor relapse be controlled again with the 1st line treatment, or is the tumor resistant thereto
c) whether a tumor relapse after 1st line treatment can be controlled again with a 2nd line treatment, and if so, which one

**Figures**

Fig 1: Dose response curves for Taxol (TAX), Staurosporine (STA) and Doxorubicine (DOX) after 5 days exposure based on size measurements. The one-fifth inhibitory concentration (IC20), or half maximal inhibitory concentration (IC50), respectively, is a measure of the effectiveness of a substance in inhibiting a specific biological or biochemical function, in this case the viability of the tumor microtissue. The following concentrations were used:

**Table 3**

|  | TAX: | STA: | DOX: |
|---|---|---|---|
| IC20: | 6.25 nM | 31.25 nM | 50 nM |
| IC50: | 12.5 nM | 62.5 nM | 200 nM |

he maximum response concentration is the lowest concentration derived from the dose response curve which leads to a maximum effect on either size and/or intra-tissue ATP value.

Fig. 2: Size comparison of HCT116 microtissues treated for 10 days with taxol (TAX), doxorubicin (DOX) and staurosporine (STA) (continuous exposure) compared to cultures where drug exposure was stopped after 5 days (marked by an asterisk). Growth is calculated via the area over time in [$\mu m^2$]. 3D InSight microtissue size profiling has been carried out as described elsewhere herein. The images have been acquired with the Cell[3]iMager manufactured by, Insphero AG, Schlieren, CH. The solid line around the tissues marks their perimeter has been determined by said device. On that basis, other size parameters, like diameter, volume or area of the optical cross section can be determined.

Fig. 3: Size profiling over time with HCT116 microtissues treated with different concentrations of taxol (TAX), doxorubicin (DOX) and staurosporine (STA) after a 10-days continuous treatment and removal of the drug after 5 days (images marked with*). Dose response is clearly visible for all drugs tested.

Fig. 4: ATP-content of HCT116 microtissues after drug exposure over 10 days with taxol, doxorubicin and staurosporine compared with the ATP-content of microtissues which weren't treated with the drug after day 5 (marked by an asterisk).

Fig 5: Growth profiles over 10 days in culture for Taxol (TAX), Staurosporine (STA) and Doxorubicine (DOX) using their respective IC20 concentrations determined from the dose response curves. Growth is displayed as tumor volume over

time [$\mu m^3$]. The control group is treated with the vehicle control (DMSO), in the pulsed group the compound was removed after 5 days and in the max response group the lowest concentration of the maximum response was used.

Fig. 6: Growth profiles over 10 days in culture for Taxol (TAX), Staurosporine (STA) and Doxorubicine (DOX) using their respective IC50 concentrations determined from the dose response curves. Growth is displayed as tumor volume over time [$\mu m^3$]. The control group is treated with the vehicle control (DMSO), in the pulsed group the compound was removed after 5 days and in the max response group the lowest concentration of the maximum response was used.

Fig. 7: Schematic to show how different parameters are determined.

To determine time to tumor relapse, the cell proliferation and/or viability of at least one 3-dimensional cell culture or tissue which has been exposed to the at least one anti-cancer agent in an interrupted fashion ("pulsed treatment group"), and the cell proliferation and/or viability of at least one 3-dimensional cell culture or tissue which has been exposed to at least one anti-cancer agent without any interruption ("continuous treatment group") are monitored. Then, a time after onset of exposure to the anti-cancer agent is determined at which said quotient exceeds a given threshold (kinetic measurement). Such tumor relapse time can for example be defined as the time at which the cell culture or tissue that underwent interrupted exposure has gained, e.g., 10 % size increase compared to the cell culture or tissue that underwent interrupted exposure.

To determine a growth constant of at least one 3-dimensional cell culture or tissue, a quotient of size (e.g., diameter, volume or area of an optical cross section) over time after treatment onset can for example be created. In such way, growth constants for the continuous treatment group, the pulsed treatment group and the untreated control group can be formed.

To determine a tumor relapse index, the sizes (e.g., diameter, volume or area of an optical cross section) of at least one 3-dimensional cell culture or tissue in the continuous treatment group, the pulsed treatment group and the untreated control group are determined at a given point of time (endpoint measurement).

Then,

(i) the difference (e.g., $d_2$) between the size of at least one 3-dimensional cell culture or tissue in the continuous treatment group and at least one 3-dimensional cell culture or tissue in the pulsed treatment group is formed, and

(ii) the difference (e.g., $d_1$) between the size of at least one 3-dimensional cell culture or tissue in the pulsed treatment group and at least one 3-dimensional cell culture or tissue in the untreated control group is formed.

Then, a quotient is formed out of the two differences (e.g., $d_1/d^2$) to deliver a tumor relapse index. Such tumor relapse indices can be determined for different inhibitory concentration of the respective anti-cancer agents.

Fig. 8: Overview of the relative tumor relapse indices (ReII) of Taxol, Staurosporine and Doxorubicin of $IC_{20}$ and $IC_{50}$ concentrations. Underlying data are shown in Figs 6 ($IC_{50}$) and 5 ($IC_{20}$).

In Figs 8, 6 and 5 it can be seen that, while at $IC_{20}$, Taxol, Staurosporine and Doxorubicin have similar tumor relapse indices, Taxol has a much higher tumor relapse index at IC50 than the two others.

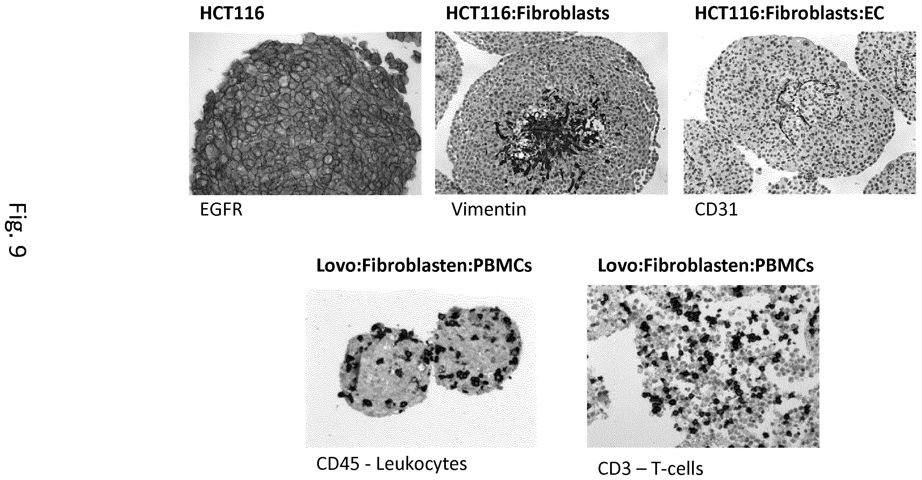

Fig. 9 Homotypic and heterotypic tumor models based on HCT116 colorectal cancer cells. HCT116 microtissue can for example be generated

a) in the presence of fibroblasts (as shown in brown by staining Vimentin),
b) in the presence of fibroblasts and endothelial cells (EC) (as shown in brown by staining CD31),
c) in the presence of migrating leucocytes (as shown in brown by staining CD45) and
d) in the presence of migrating T-cells (as shown in brown by staining CD3).

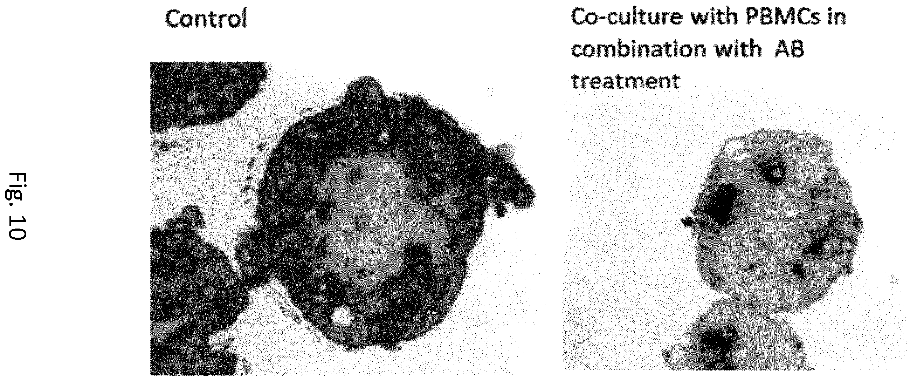

Fig. 10: Effect of an immune checkpoint inhibitor (anti CEA-IL2v) on a 3-dimensional cell tissue comprising tumor cells derived from a colon cancer (peripheral layers, in brown) and stroma fibroblasts (core region). The tissue was co-cultured with PBMC (Peripheral Blood Mononuclear Cells). The antibody treatment caused PBMC-mediated cell death. Cell killing was tumor specific as shown by removal of the outer cancer cell population (shown in brown). The stroma core region was not affected.

**Claims**

1. An *in vitro* tumor relapse assay method, which method comprises:

a') Producing a 3-dimensional cell culture or tissue in a hanging drop culture system or a low adhesion well culture system,

a) Providing a 3-dimensional cell culture or tissue comprising immortalized, malignant, cancerous and/or neoplastic cells,

b) Exposing the 3-dimensional cell culture or tissue to at least one anti-cancer agent,

c) Interrupting the exposure,

b') Exposing the 3-dimensional cell culture or tissue at least one further time with the same or at least one different anti-cancer agent,

d) Determining the effect of said exposure and interruption on the 3-dimensional cell culture or tissue.

2. The tumor relapse assay method according to any of the aforementioned claims, in which method the 3-dimensional cell culture or tissue also comprises at least one cell type selected from the group consisting of:

- Immune cells
- Stroma cells
- Fibroblasts
- Cancer stem cells, and/or
- Immortalized, malignant, cancerous and/or neoplastic cells that are known to be resistant or sensitive to a given anti-cancer agent.

3. The tumor resistance assay method according to any of the aforementioned claims, in which method the one or more anti-cancer-agents are selected from the group consisting of:

- cytotoxic and/or chemotherapeutic agents
- targeted drugs
- immunotherapeutic agents
- radiation

and/or combinations thereof.

4. The tumor relapse assay method according to any of the aforementioned claims, in which method the determination of cell proliferation and/or cell viability is at least one selected from the group consisting of:

- Size determination of the 3-dimensional cell culture or tissue
- Quantification of internal reporter gene expression
- Determination of the intracellular ATP content.
- Determination of particular biomarkers

5. The tumor relapse assay method according to claim 4, in which method the size determination of the 3-dimensional cell culture or tissue refers to at least one parameter selected from the group consisting of:

- Diameter
- perimeter
- volume
- area of an optical cross section

6. The tumor relapse assay method according to any of the aforementioned claims, wherein the determination of the effect of the anti-cancer agent exposure on the 3-dimensional cell culture or tissue is determined by

- kinetic measurements of cell proliferation and/or cell viability, and /or
- endpoint measurements of cell proliferation and/or cell viability.

7. The tumor relapse assay method according to any of the aforementioned claims, in which method the cell proliferation and/or viability of the 3-dimensional cell culture or tissue which has been exposed to the at least one anti-cancer agent in an interrupted fashion ("pulsed treatment group") is compared to

- the cell proliferation and/or viability of at least one 3-dimensional cell culture or tissue which has been exposed to at least one anti-cancer agent without any interruption ("continuous treatment group"), and/or
- the cell proliferation and/or viability of at least one 3-dimensional cell culture or tissue which has not been exposed to the at least one anti-cancer agent ("untreated control group").

8. The tumor relapse assay method according to any of the aforementioned claims, which method comprises the determination of a time to tumor relapse by forming a quotient of:

• the cell proliferation and/or viability of at least one 3-dimensional cell culture or tissue which has been exposed to the at least one anti-cancer agent in an interrupted fashion ("pulsed treatment group"), and
• the cell proliferation and/or viability of at least one 3-dimensional cell culture or tissue which has been exposed to at least one anti-cancer agent without any interruption,

and determining a time after onset of exposure to the anti-cancer agent at which said quotient exceeds a given threshold.

9. The tumor relapse assay method according to any of the aforementioned claims, which method comprises the determination of a tumor relapse index.

10. The tumor relapse assay method according to claim 9, in which method the determination of a tumor relapse index comprises the following steps:

a) determine the cell proliferation and/or viability of at least

(i) one 3-dimensional cell culture or tissue which has been exposed to the at least one anti-cancer agent in an interrupted fashion ("pulsed treatment group"),
(ii) one 3-dimensional cell culture or tissue which has been exposed to the at least one anti-cancer agent without any interruption ("continuous treatment group"), and
(iii) one 3-dimensional cell culture or tissue which has not been exposed to the at least one anti-cancer agent ("untreated control group")

at a given point of time,
b) determine

(i) the difference (e.g., d2) between the cell proliferation and/or viability of at least one 3-dimensional cell culture or tissue in the continuous treatment group and at least one 3-dimensional cell culture or tissue in the pulsed treatment group, and
(ii) the difference (e.g., $d_2$) between the cell proliferation and/or viability of at least one 3-dimensional cell culture or tissue in the pulsed treatment group and at least one 3-dimensional cell culture or tissue in the untreated control group, and

c) form a quotient out of the two differences (e.g., $d_1/d_2$) to deliver a tumor relapse index.

11. The tumor relapse assay method according to any of the aforementioned claims, wherein the determination of the cell proliferation and/or viability of a 3-dimensional cell culture or tissue treated in a particular manner comprises the determination of the cell proliferation and/or viability of two or more 3-dimensional cell cultures or tissues treated in the same manner, and forming a statistical mean or median for proliferation and/or viability.

12. The tumor relapse assay method according to any of the aforementioned claims, which method further comprises a step of determining an inhibitory concentration of the at least one anti-cancer agent on the 3-dimensional cell culture or tissue.

13. The tumor relapse assay method according to any of the aforementioned claims, in which method

• at least two 3-dimensional cell cultures or tissues of similar or identical composition are used in such way that each cell culture or tissue is exposed to a different anti-cancer agent, or
• at least two 3-dimensional cell cultures or tissues of different composition are used in such way that both cell cultures or tissues are exposed to the same anti-cancer agent.

14. The tumor relapse assay method according to any of the aforementioned claims, which method further comprises exposure of the 3-dimensional cell culture or tissue to one or more immune cells.

15. Use of a tumor relapse assay method according to any of the aforementioned claims for at least one purpose selected from the group consisting of:

> • comparing different anti-cancer agents, and the respective risks of relapses,
> • screening drug libraries in search of anti-cancer agents with low risk of becoming subject of a relapse,
> • screening drug libraries in search of anti-cancer agents which have efficacy against a tumor that has already relapsed after therapy with another anti-cancer agent,
> • screening drug libraries in search of anti-cancer agents which restore the sensitivity of a tumor that has already relapsed after therapy with another anti-cancer agent,
> • finding a patient-specific anti-cancer therapy,
> • creating benchmarks for particular anti-cancer drugs regarding their risk to become subject of relapses,
> • comparing different 3-dimensional cell cultures or tissues on their potential to develop relapses after therapy with an anti-cancer agent, and/or
> • investigating the reasons for tumor relapse after therapy with an anti-cancer agent.

**Patentansprüche**

1. Ein *in vitro* Tumor-Rückfall-Assay, aufweisend:

   a') Produzieren einer 3-dimensionalen Zellkultur oder Gewebe in einem hangingdrop-Kultursystem oder einem low-adhesion-well-Kultursystem,
   a) Bereitstellen einer 3-dimensionalen Zellkultur oder Gewebe aufweisend immortalisierte, maligne, krebsartige und/oder neoplastische Zellen,
   b) Exponieren der 3-dimensionalen Zellkultur oder Gewebe zu mindestens einem Antikrebswirkstoff,
   c) Unterbrechen der Exposition,
   b') Exponieren der 3-dimensionalen Zellkultur oder Gewebe mindestens ein weiteres Mal mit dem selben oder mindestens einem unterschiedlichen Antikrebswirkstoff,
   d) Bestimmen des Effekts von besagter Exposition und Unterbrechung auf die 3-dimensionale Zellkultur oder Gewebe.

2. Tumor-Rückfall-Assay nach einem der vorherigen Ansprüche, bei dem die 3-dimensionale Zellkultur oder Gewebe ebenfalls einen Zelltyp aufweist, ausgewählt aus der Gruppe bestehend aus:

   > • Immunzellen
   > • Stromazellen
   > • Fibroblasten
   > • Krebsstammzellen, und/oder
   > • Immortalisierte, maligne, krebsartige und/oder neoplastische Zellen, die bekannt sind resistent oder sensitiv für einen bestimmten Antikrebswirkstoff zu sein.

3. Tumor-Rückfall-Assay nach einem der vorherigen Ansprüche, bei dem ein oder mehrere Antikrebswirkstoffe ausgewählt werden aus der Gruppe bestehend aus:

   > • zytotoxische und/oder chemotherapeutische Wirkstoffe
   > • gezielte Medikamente
   > • immuntherapeutische Wirkstoffe
   > • Bestrahlung

   und/oder Kombinationen davon.

4. Tumor-Rückfall-Assay nach einem der vorherigen Ansprüche, bei dem die Bestimmung der Zellproliferation und/oder der Zellviabilität mindestens eines ausgewählt aus der Gruppe bestehend aus:

   > • Größenbestimmung der 3-dimensionalen Zellkultur oder Gewebe
   > • Quantifizierung interner Reportergenexpression
   > • Bestimmung des intrazellulären ATP Gehalts
   > • Bestimmung von bestimmten Biomarkern.

ist.

5. Tumor-Rückfall-Assay nach Anspruch 4, bei dem sich die Größenbestimmung der 3-dimensionalen Zellkultur oder Gewebe auf mindestens einen Parameter bezieht, ausgewählt aus der Gruppe bestehend aus:

- Durchmesser
- Umfang
- Volumen
- Fläche eines optischen Querschnitts.

6. Tumor-Rückfall-Assay nach einem der vorherigen Ansprüche, wobei die Bestimmung des Effekts der Antikrebswirkstoff Exposition auf die 3-dimensionale Zellkultur oder Gewebe bestimmt wird durch

- kinetische Messungen der Zellproliferation und/oder Zellviabilität, und/oder
- Endpunktmessungen der Zellproliferation und/oder Zellviabilität.

7. Tumor-Rückfall-Assay nach einem der vorherigen Ansprüche, bei dem die Zellproliferation und/oder Viabilität der 3-dimensionalen Zellkultur oder Gewebe, welche mindestens einem Antikrebswirkstoff in unterbrechender Weise ausgesetzt wurde ("pulsierte Behandlungsgruppe"), verglichen wird mit

- der Zellproliferation und/oder Viabilität von mindestens einer 3-dimensionalen Zellkultur oder Gewebe, welche mindestens einem Antikrebswirkstoff ohne jegliche Unterbrechung ausgesetzt wurde ("kontinuierliche Behandlungsgruppe"), und/oder
- der Zellproliferation und/oder Viabilität von mindestens einer 3-dimensionalen Zellkultur oder Gewebe, welche nicht mindestens einem Antikrebswirkstoff ausgesetzt wurde ("unbehandelte Kontrollgruppe").

8. Tumor-Rückfall-Assay nach einem der vorherigen Ansprüche, aufweisend die Bestimmung einer Zeit für den Tumorrückfall durch die Bildung eines Quotienten aus

- der Zellproliferation und/oder Viabilität von mindestens einer 3-dimensionalen Zellkultur oder Gewebe, welche mindestens einem Antikrebswirkstoff in unterbrechender Weise ausgesetzt wurde ("pulsierte Behandlungsgruppe"), und
- der Zellproliferation und/oder Viabilität von mindestens einer 3-dimensionalen Zellkultur oder Gewebe, welche mindestens einem Antikrebswirkstoff ohne jegliche Unterbrechung ausgesetzt wurde,

und Bestimmen einer Zeit nach Beginn der Exposition zu mindestens einem Antikrebswirkstoff, bei der der besagte Quotient eine bestimmte Schwelle übersteigt.

9. Tumor-Rückfall-Assay nach einem der vorherigen Ansprüche, aufweisend die Bestimmung von einem Tumor-Rückfallindex

10. Tumor-Rückfall-Assay nach Anspruch 9, bei dem die Bestimmung von einem Tumor-Rückfallindex die folgenden Schritte aufweist:

a) bestimmen der Zellproliferation und/oder Viabilität von mindestens

(i) einer 3-dimensionale Zellkultur oder Gewebe, welche mindestens einem Antikrebswirkstoff in unterbrechender Weise ausgesetzt wurde ("pulsierte Behandlungsgruppe"),
(ii) einer 3-dimensionalen Zellkultur oder Gewebe, welche mindestens einem Antikrebswirkstoff ohne jegliche Unterbrechung ausgesetzt wurde ("kontinuierliche Behandlungsgruppe"), und
(iii) einer 3-dimensionalen Zellkultur oder Gewebe, welche nicht mindestens einem Antikrebswirkstoff ausgesetzt wurde ("unbehandelte Kontrollgruppe")

an einem bestimmten Zeitpunkt,
b) bestimmen

(i) des Unterschieds (z.B., $d_2$) zwischen der Zellproliferation und/oder Viabilität von mindestens einer 3-dimensionalen Zellkultur oder Gewebe in der kontinuierlichen Behandlungsgruppe und mindestens einer

17

3-dimensionalen Zellkultur oder Gewebe in der pulsierten Behandlungsgruppe, und

(ii) des Unterschieds (z.B., $d_2$) zwischen der Zellproliferation und/oder Viabilität von mindestens einer 3-dimensionalen Zellkultur oder Gewebe in der pulsierten Behandlungsgruppe und mindestens einer 3-dimensionalen Zellkultur oder Gewebe in der unbehandelten Kontrollgruppe, und

c) bilden eines Quotienten aus den beiden Unterscheiden (z.B., $d_1/d_2$) um einen Tumor-Rückfallindex zu liefern.

11. Tumor-Rückfall-Assay nach einem der vorherigen Ansprüche, wobei die Bestimmung der Zellproliferation und/oder Viabilität einer 3-dimensionalen Zellkultur oder Gewebe, behandelt in einer bestimmten Weise, die Bestimmung der Zellproliferation und/oder Viabilität von zwei oder mehr 3-dimensionalen Zellkulturen oder Geweben, behandelt in derselben Weise, und das Bilden eines statistischen Mittelwerts oder Medians für die Proliferation und/oder Viabilität aufweist.

12. Tumor-Rückfall-Assay nach einem der vorherigen Ansprüche, ferner aufweisend einen Schritt zur Bestimmung einer inhibitorischen Konzentration von mindestens einem Antikrebswirkstoff auf die 3-dimensionale Zellkultur oder Gewebe.

13. Tumor-Rückfall-Assay nach einem der vorherigen Ansprüche, bei dem

• mindestens zwei 3-dimensionale Zellkulturen oder Gewebe mit ähnlicher oder identischer Zusammensetzung so genutzt werden, dass jede Zellkultur oder Gewebe einem unterschiedlichen Antikrebswirkstoff ausgesetzt wird, oder

• mindestens zwei 3-dimensionale Zellkulturen oder Gewebe mit unterschiedlicher Zusammensetzung so genutzt werden, dass jede Zellkultur oder Gewebe demselben Antikrebswirkstoff ausgesetzt wird.

14. Tumor-Rückfall-Assay nach einem der vorherigen Ansprüche, ferner aufweisend die Exposition der 3-dimensionalen Zellkultur oder Gewebe zu einer oder mehreren Immunzellen.

15. Verwendung eines Tumor-Rückfall-Assays nach einem der vorherigen Ansprüche für mindestens einen Zweck, ausgewählt aus der Gruppe bestehend aus:

• Vergleichen von unterschiedlichen Antikrebswirkstoffen, und dem entsprechenden Risiko von Rückfällen

• Screening von Medikamentensammlungen auf der Suche nach Antikrebswirkstoffen mit geringem Risiko Gegenstand eines Rückfalls zu werden,

• Screening von Medikamentensammlungen auf der Suche nach Antikrebswirkstoffen, die wirksam gegen einen Tumor, der bereits rückfällig nach der Behandlung mit einem anderen Antikrebswirkstoff geworden ist, sind,

• Screening von Medikamentensammlungen auf der Suche nach Antikrebswirkstoffen, die die Sensitivität eines Tumors, der bereits rückfällig nach der Behandlung mit einem anderen Antikrebswirkstoff geworden ist, wiederherstellen.

• Finden einer patienten-spezifischen Antikrebs Therapie,

• Erstellen von Richtwerten für bestimmte Antikrebsmedikamente in Bezug auf deren Risiko Gegenstand von Rückfällen zu werden,

• Vergleichen von verschiedenen 3-dimensionalen Zellkulturen oder Geweben auf deren Potential Rückfälle nach der Behandlung mit einem Antikrebswirkstoff zu entwickeln, und/oder

• Untersuchen der Ursachen für Tumorrückfall nach der Therapie mit einem Antikrebswirkstoff.

**Revendications**

1. Procédé de dosage d'une récidive de tumeur *in vitro,* lequel procédé comprend :

a') la production d'un tissu ou d'une culture cellulaire tridimensionnelle dans un système de culture en gouttes suspendues ou un système de culture en alvéoles à faible adhérence,

a) la fournissant d'un tissu ou d'une culture cellulaire tridimensionnelle comprenant des cellules immortalisées, malignes, cancéreuses et/ou néoplasiques,

b) l'exposition du tissu ou de la culture cellulaire tridimensionnelle à au moins un agent anticancéreux,

c) l'interruption de l'exposition,

b') l'exposition du tissu ou de la culture cellulaire tridimensionnelle à au moins un temps supplémentaire avec le même ou au moins un agent anticancéreux différent,
d) la détermination de l'effet desdites exposition et interruption sur le tissu ou la culture cellulaire tridimensionnelle.

2. Procédé de dosage d'une récidive de tumeur selon l'une quelconque des revendications précédentes, procédé dans lequel le tissu ou la culture cellulaire tridimensionnelle comprend également au moins un type de cellule sélectionné dans le groupe constitué par :

- ○ des cellules immunitaires
- ○ des cellules de Stroma
- ○ des fibroblastes
- ○ des cellules-souches cancéreuses, et/ou
- ○ des cellules immortalisées, malignes, cancéreuses et/ou néoplasiques que l'on connaît comme étant résistantes ou sensibles à un agent anticancéreux donné.

3. Procédé de dosage de résistance tumorale selon l'une quelconque des revendications précédentes, procédé dans lequel le ou les plusieurs agents anticancéreux sont sélectionnés dans le groupe constitué par :

- ○ des agents cytotoxiques et/ou chimiothérapeutiques
- ○ des médicaments ciblés
- ○ des agents immunothérapeutiques
- ○ un rayonnement
- et/ou des combinaisons de ceux-ci.

4. Procédé de dosage d'une récidive de tumeur selon l'une quelconque des revendications précédentes, procédé dans lequel la détermination de prolifération cellulaire et/ou de viabilité cellulaire est au moins une sélectionnée dans le groupe constitué par :

- ○ la détermination de taille du tissu ou de la culture cellulaire tridimensionnelle
- ○ la quantification de l'expression de gène rapporteur interne
- ○ la détermination du contenu ATP intracellulaire
- ○ la détermination de marqueurs biologiques particuliers

5. Procédé de dosage d'une récidive de tumeur selon la revendication 4, procédé dans lequel la détermination de taille du tissu ou de la culture cellulaire tridimensionnelle se réfère à au moins un paramètre sélectionné dans le groupe constitué par :

- ○ le diamètre
- ○ le périmètre
- ○ le volume
- ○ la surface d'une section transversale optique.

6. Procédé de dosage d'une récidive de tumeur selon l'une quelconque des revendications précédentes, dans lequel la détermination de l'effet de l'exposition à l'agent anticancéreuse sur le tissu ou la culture cellulaire tridimensionnelle est déterminée par

- ○ des mesures cinétiques de prolifération cellulaire et/ou de viabilité cellulaire, et/ou
- ○ des mesures de but final de prolifération cellulaire et/ou de viabilité cellulaire.

7. Procédé de dosage d'une récidive de tumeur selon l'une quelconque des revendications précédentes, procédé dans lequel la viabilité et/ou la prolifération cellulaire du tissu ou de la culture cellulaire tridimensionnelle qui a été exposé(e) à l'au moins un agent anticancéreux d'une façon interrompue (« groupe de traitement pulsé ») est comparée à

- ○ la viabilité et/ou la prolifération cellulaire d'au moins un tissu ou une culture cellulaire tridimensionnelle qui a été exposé(e) à au moins un agent anticancéreux sans aucune interruption (« groupe de traitement continu »), et/ou
- ○ la viabilité et/ou la prolifération cellulaire d'au moins un tissu ou une culture cellulaire tridimensionnelle qui n'a pas été exposé(e) à au moins un agent anticancéreux (« groupe témoin non traité »).

8.  Procédé de dosage d'une récidive de tumeur selon l'une quelconque des revendications précédentes, lequel procédé comprend la détermination d'un temps à une récidive de tumeur en formant un quotient de :

    ∘ la viabilité et/ou la prolifération cellulaire d'au moins un tissu ou une culture cellulaire tridimensionnelle qui a été exposé(e) à au moins un agent anticancéreux d'une façon interrompue (« groupe de traitement pulsé »), et
    ∘ la viabilité et/ou la prolifération cellulaire d'au moins un tissu ou une culture cellulaire tridimensionnelle qui a été exposé(e) à au moins un agent anticancéreux sans aucune interruption,

    et la détermination d'un temps après le début d'exposition à l'agent anticancéreux auquel ledit quotient dépasse un seuil donné.

9.  Procédé de dosage d'une récidive de tumeur selon l'une quelconque des revendications précédentes, lequel procédé comprend la détermination d'un indice de récidive de tumeur.

10. Procédé de dosage d'une récidive de tumeur selon la revendication 9, procédé dans lequel la détermination d'un indice de récidive de tumeur comprend les étapes suivantes :

    a) déterminer la viabilité et/ou la prolifération cellulaire d'au moins

       (i) un tissu ou une culture cellulaire tridimensionnelle qui a été exposé (e) à au moins un agent anticancéreux d'une façon interrompue (« groupe de traitement pulsé »),
       (ii) un tissu ou une culture cellulaire tridimensionnelle qui a été exposé (e) à au moins un agent anticancéreux sans aucune interruption (« groupe de traitement continu »), et
       (iii) un tissu ou une culture cellulaire tridimensionnelle qui n'a pas été exposé (e) à au moins un agent anticancéreux (« groupe témoin non traité »)

    à un point de temps donné,
    b) déterminer

       (i) la différence (par exemple, d2) entre la viabilité et/ou la prolifération cellulaire d'au moins une culture cellulaire tridimensionnelle dans le groupe de traitement continu et au moins un tissu ou une culture cellulaire tridimensionnelle dans le groupe de traitement pulsé, et
       (ii) la différence (par exemple, $d_2$) entre la viabilité et/ou la prolifération cellulaire d'au moins un tissu ou une culture cellulaire tridimensionnelle dans le groupe de traitement pulsé et au moins un tissu ou une culture cellulaire tridimensionnelle dans le groupe témoin non traité, et

    c) former un quotient des deux différences (par exemple, $d_1/d_2$) pour délivrer un indice de récidive de tumeur.

11. Procédé de dosage d'une récidive de tumeur selon l'une quelconque des revendications précédentes, dans lequel la détermination de la viabilité et/ou de la prolifération cellulaire d'un tissu ou d'une culture cellulaire tridimensionnelle traité(e) d'une façon particulière comprend la détermination de la viabilité et/ou de la prolifération cellulaire de deux ou plusieurs tissus ou cultures cellulaires tridimensionnelles traité(e)s de la même façon, et la formation d'une moyenne statistique ou médiane pour la prolifération et/ou la viabilité.

12. Procédé de dosage d'une récidive de tumeur selon l'une quelconque des revendications précédentes, lequel procédé comprend en outre une étape de détermination d'une concentration inhibitrice de l'au moins un agent anticancéreux sur le tissu ou la culture cellulaire tridimensionnelle.

13. Procédé de dosage d'une récidive de tumeur selon l'une quelconque des revendications précédentes, procédé dans lequel
    ∘ au moins deux tissus ou cultures cellulaires tridimensionnelles de composition similaire ou identique sont utilisé(e)s d'une telle façon que chaque tissu ou culture cellulaire est exposé(e) à un agent anticancéreux différent, ou
    ∘ au moins deux tissus ou cultures cellulaires tridimensionnelles de composition différente sont utilisé(e)s d'une telle façon que les deux tissus ou cultures cellulaires sont exposé(e)s au même agent anticancéreux.

14. Procédé de dosage d'une récidive de tumeur selon l'une quelconque des revendications précédentes, lequel procédé comprend en outre l'exposition du tissu ou de la culture cellulaire tridimensionnelle à une ou plusieurs cellules immunitaires.

15. Utilisation d'un procédé de dosage d'une récidive de tumeur selon l'une quelconque des revendications précédentes pour au moins un but sélectionné dans le groupe constitué par :

    ◦ la comparaison d'agents anticancéreux différents et des risques respectifs de récidives,
    ◦ l'examen de banques de médicaments à la recherche d'agents anticancéreux avec un faible risque de devenir sujet à une récidive,
    ◦ l'examen de banques de médicaments à la recherche d'agents anticancéreux qui ont une efficacité contre une tumeur qui a déjà récidivé après une thérapie avec un autre agent anticancéreux,
    ◦ l'examen de banques de médicament à la recherche d'agents anticancéreux qui rétablissent la sensibilité d'une tumeur qui a déjà récidivé après une thérapie avec un autre agent anticancéreux,
    ◦ la découverte d'une thérapie anticancéreuse spécifique au patient,
    ◦ la création de points de référence pour des médicaments anticancéreux particuliers quant à leur risque de devenir sujet à des récidives,
    ◦ la comparaison de tissus ou de cultures cellulaires tridimensionnelles différentes en ce qui concerne leur potentiel à développer des récidives après une thérapie avec un agent anticancéreux, et/ou
    ◦ l'investigation des raisons de récidive de tumeur après une thérapie avec un agent anticancéreux.

Fig. 1

Fig. 2

TX

Control    6 nM*    50

Control    6 nM    50 nM

DOX

Control    50 nM*    100 nM*

Control    50 nM    100 nM

STA

Control    125 nM*    500 nM*

Control    125 nM    500 nM

Fig. 3

Fig. 4

Fig. 5

Fig. 6

Fig. 7

Size

$$ReI_{IC20} = \frac{d_1}{d_2}$$

$$ReI_{IC50} = \frac{d_1}{d_2}$$

Untreated
control group

$d_1$

$k_{control}$

Pulsed treatment
group

$k_{relapse}$

$d_2$

10%

$k_{continous}$

Continuous
treatment group

Tumor relapse time (10% size
difference after compound pulse)

Rel: Relapse Index

K: growth constant $[\frac{\mu m^3}{day}]$

| | Taxol | | Staurosporine | | Doxorubicine | |
|---|---|---|---|---|---|---|
| | IC20 | IC50 | IC20 | IC50 | IC20 | IC50 |
| d1 [$\mu$m$^3$] | 55241 | 139110 | 43488 | 52383 | 36223 | 100935 |
| d2 [$\mu$m$^3$] | 92935 | 9066 | 102444 | 93550 | 111968 | 47256 |
| **Rel** | **0.59** | **15.3** | **0.42** | **0.56** | **0.32** | **2.1** |

Rel =10 - ∞: Low probability of tumor recurrence and high impact on tumor growth rate at a given concentration and treatment scheme

Rel = 1-10: Moderate probability of tumor recurrence and/or reduced tumor growth rate at a given concentration and treatment scheme

Rel = <1:  High probability of tumor recurrence and/or minor impact on tumor growth at a given concentration and treatment scheme

Fig. 8

Fig. 9

Fig. 10

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **MAS et al.** *Journal of Biotechnology,* vol. 205, 111-119

- **VINCI et al.** Advances in establishment and analysis of three-dimensional tumor spheroid-based functional assays for target validation and drug evaluation. *BMC BIOLOGY,* vol. 10 (1), 29